# EUROPEAN PATENT APPLICATION

(11) **EP 0 781 564 A2**
(43) Date of publication of application: **02.07.1997**
(21) Application number: 96309355.4
(22) Date of filing: 20.12.1996
(51) Int. Cl.: A61L 27/00, A61L 31/00, A61L 17/00, A61L 15/32

(54) **Material for medical use comprising human-derived natural collagen membrane**

(30) Priority: 21.12.1995 JP 333182/95; 09.10.1996 JP 268440/96
(71) Applicant: SHIMIZU, Yasuhiko, Uji-shi, Kyoto 611 (JP); AMD Ltd., Shibuya-ku, Tokyo (JP)
(72) Inventor: Shimizu, Yasuhiko, Uji-shi, Kyoto (JP)
(74) Representative: Watkins, David

(57) **Abstract**

Disclosed are a material for medical use, which comprises at least 2 layers of a human-derived natural collagen membrane, wherein the layers of the human-derived natural collagen membrane are adhered with an adhesive, a wound dressing material, a suture-reinforcement material, a visceral wound prosthetic material and an adhesion-preventing material comprising the same, and a process for preparing the same.

## Description

### BACKGROUND OF THE INVENTION

The present invention relates to a material for medical use, which comprises a human-derived natural collagen membrane, and in particular, relates to a material for medical use, which comprises a human-derived natural collagen membrane and is useful as a wound dressing material, a suture-reinforcement material for an autosuture or a visceral wound prosthetic material.

As a living body-derived material for medical use which is variously used in the field of medicine, various materials have been widely used outside or inside a living body. As a material used outside a living body, a collagen material which is obtained from various collagen-containing raw materials, or a lyophilized swine dermis has been used as, for example, a wound dressing material. As a material used inside a living body, a bovine or swine-derived cardiac valve, an equine pericardium, a bovine pericardium, a human dura or a human fascia lata has been used for various purposes.

When living body tissues are injured or when a living body is subjected to a surgical operation, various living body-derived materials have been used as a wound dressing material in order to protect a wound and prevent an infection of the wound and accelerate the healing of the wound.

As a wound dressing material, a collagen material or a lyophilized swine dermis has been used. Collagen which can be obtained from various animals is excellent in the affinity for a living body and the tissue compatibility, has a low antigenicity, has an accelerating activity on the extension and growth of host cells to induce the regeneration of tissues, and also has a hemostatic activity. From a raw material which is obtained from various animals and contains much collagen, a collagen material is obtained by solubilizing the collagen with an acid, an alkali or an enzyme. Such a collagen material is used singly or as a combination material in which a collagen layer is used together with a synthetic high molecular material (for control of moisture evaporation and for prevention of infection) or used together with a biodegradable material.

As a material for medical use in which a collagen layer is used together with a synthetic high molecular material, a material in which a collagen layer is covered with a layer comprising a synthetic high molecular material such as silicone and polyurethane has been used (Matsuda, K. et al.: Biomaterials 1990, 11, 351-355, Suzuki, S. et al.: Biomaterials 1990, 11, 356-360).

As a material for medical use in which a collagen layer is used together with a biodegradable material, a material in which a collagen layer is covered with a material comprising polyglycolic acid (PGA), a copolymer of glycolic acid and lactic acid, or a mixture of polyglycolic acid and polylactic acid (PLA) is presently under development, but has not yet been put to practical use (Japanese Provisional Patent Publication No. 292716/1994).

Japanese Provisional Patent Publication No. 56987/1993 discloses that a V-type collagen-like membrane material which is obtained from a human fetal membrane without destroying a membrane structure as a natural collagen membrane which is not reconstituted is used as a wound prosthetic material.

On the other hand, as an example of the use of a living body-derived material inside a living body, a bovine pericardium has been used as a suture-reinforcement material for an autosuture. Recently, a stapler-like autosuture made of stainless steel or polylactic acid has been used in place of hand-suture, for excision and suture of a viscus. When such an autosuture is used, blood or air sometimes leaks through a suture line after suture. Particularly in the case of a surgical operation of severe pulmonary emphysema or in the case of pulmonary emphysema-crush surgery, air-leakage sometimes continues at the autosuture-applied area. In order to prevent such air-leakage, a pipe-like dense membrane is sutured together at the suture area as a suture-reinforcement material for an autosuture. Presently, a collagen membrane derived from a bovine pericardium or a PGA non-woven fabric has been used as a suture-reinforcement material (Cooper, J.D.: Ann. Thorac. Surg. 1994, 57, 1038-1039, Crosa-Dorado, V.L. et al.: Res. Surg. 1992, 4, 152-155).

On the other hand, a viscus such as lung, liver and spleen is normally covered with a collagen membrane called as tunica serosa. When such a viscus has a wound due to excision by an operation or due to an injury, a wound surface of the viscus which lacks for the tunica serosa is exposed. If the exposed surface does not receive any treatment, air or blood flows out from the lung; bile juice or blood from the liver; and blood from the spleen. In order to prevent this, the viscus is sutured, but it is often difficult to suture the viscus.

However, a solubilized collagen material as described above has problems that it is difficult to form a material having some mechanical strength only from the solubilized collagen material, and that after the collagen material is applied, it will dissolve immediately. A lyophilized swine dermis which is a heterologous material has an antigenicity and does not have an accelerating effect on the healing of a wound. When it is used as a wound dressing material, it only reduces a pain. A living body-derived material used inside a living body such as a bovine or swine-derived cardiac valve, an equine pericardium and a bovine pericardium, which also has an antigenicity, is made non-biodegradable by subjecting it to a crosslinking treatment. Therefore, when it remains in a body for a long time, it is calcified. Since a human dura and a human fascia lata are expected to be decomposed and absorbed in a living body, they are not crosslinked. However, it is possible that they are collected from a person having a disease and then it may possibly cause an infection. Also, they are collected from a dead body, there may exist an ethical problem. Therefore, it is not certain whether or not they are safely and stably supplied in the future.

Any conventional wound dressing material comprising such a living body-derived material is intended to maintain a wound under a wet condition to accelerate the growth of living body cells. Then, it has a disadvantage that it produces a condition which is suitable for the growth of bacteria on the contrary. A wound dressing material in which a collagen layer and a synthetic high molecular material are combined is required to be exchanged several times during the treatment of a wound. Also, a certain wound dressing material causes a pain when it is peeled off from a wound, or may again injure subcutaneous tissues which are in the course of healing. Also, a wound dressing material in which a collagen layer and a biodegradable material are combined has not yet been put to practical use. A wound prosthetic material comprising a V-type collagen-like membrane material, which is a monolayer material, is like a medicinal wafer and easily tears and then it is hard to handle it.

In a suture-reinforcement material which is a pipe-like membrane comprising a bovine pericardium and is used for an autosuture, the bovine pericardium is made non-biodegradable by a crosslinking treatment due to its antigenicity. Accordingly, the suture-reinforcement material remains as a foreign matter in a living body for a long time, whereby it is calcified, which may be harmful for a living body. A suture-reinforcement material comprising a PGA non-woven fabric is biodegradable but does not have a wound-healing effect. Furthermore, since the PGA non-woven fabric is not transparent, a medical staff cannot directly see an excision area to which an autosuture is applied. Also, after the suture with the autosuture, the PGA non-woven fabric cannot be easily cut, which is dangerous.

On the other hand, as for a visceral wound as described above, development of a material for medical use has been demanded, which not only protects the wound but also accelerates the healing of the wound in the case where the wound surface of the viscus cannot be sutured.

Accordingly, there has been a demand for development of a material for medical use which not only accelerates the growth of living body cells to accelerate the healing of a wound, but also prevents bacterial growth, is decomposed after application to a living body, does not need to be exchanged, has a low antigenicity and suitable strength, is transparent, is easily handled, i.e., easily cut, and is supplied safely and stably.

### SUMMARY OF THE INVENTION

The present invention was made to solve the above problems, and an object of the present invention is to provide a material for medical use which is useful as a wound dressing material, a suture-reinforcement material for an autosuture and a visceral wound prosthetic material.

The present invention relates to a material for medical use, which comprises at least 2 layers of a human-derived natural collagen membrane, wherein said layers of the human-derived natural collagen membrane are adhered with an adhesive. The present invention also relates to a process for preparing the above material for medical use, which comprises adhering at least 2 layers of a human-derived natural collagen membrane with an adhesive to give a laminate and subjecting the laminate to a crosslinking treatment.

### DESCRIPTION OF THE PREFERRED EMBODIMENTS

The human-derived natural collagen membrane in the present invention is a collagen membrane which is collected from a human and purified and maintains the inherent membrane structure. The human-derived natural collagen membrane has a lower antigenicity as compared with living body-derived materials collected from mammals other than a human. Further, the human-derived natural collagen membrane, when applied to a wound, rapidly becomes dry to prevent bacterial proliferation. Furthermore, capillary vessels of a living body extend into the applied natural collagen membrane, whereby regeneration of living body tissues is accelerated to heal a wound soon. After application of the human-derived natural collagen membrane to a living body, it is decomposed and absorbed in a living body, or naturally drops from a wound after healing. Then, it does not need to be exchanged until the wound heals. For these reasons, the human-derived natural collagen membrane is used in the present invention.

In the present invention, as the human-derived natural collagen membrane, any collagen membrane which is human-derived and maintains the inherent membrane structure can be used. In particular, it is preferred to use an amnion-derived collagen membrane or a chorion-derived collagen membrane, which can be obtained by separating a fetal membrane from a mass consisting of a human fetal membrane, a placenta and an umbilical cord, obtained after delivery as an afterbirth; separating an amnion or a chorion from the fetal membrane; and purifying the separated amnion or chorion.

The human amnion-derived collagen membrane and the human chorion-derived collagen membrane have been safely and stably supplied as compared with a human dura or a human fascia lata, which is collected from a dead body and has been conventionally used as a human-derived material for medical use. Therefore, in the present invention, it is preferred to use the human amnion-derived collagen membrane or the human chorion-derived collagen membrane.

The human amnion-derived collagen membrane and the human chorion-derived collagen membrane may be obtained from a mass consisting of a human fetal membrane, a placenta and an umbilical cord, obtained immediately after delivery as an afterbirth by any method. For example, they can be separated and purified by the method described in Japanese Provisional Patent Publication No. 56987/1993. Namely, only a fetal membrane is separated from a mass consisting of a human fetal membrane, a placenta and an umbilical cord, obtained immediately after delivery, in a benzalkonium chloride solution or a benzalkonium bromide solution; an amnion and a chorion are peeled off from the fetal membrane which consists of 4 layers; remaining tissues on the amnion and the chorion are physically and enzymatically removed; and they are purified by ultrasonic washing to obtain a human amnion-derived collagen membrane and a human chorion-derived collagen membrane. As the enzyme, ficin is used. Since ficin used together with cysteine has a higher activity, it is preferred to use ficin together with cysteine. Also, the human amnion-derived collagen membrane and the human chorion-derived collagen membrane may be purified by the method of Brendel et al. described in Provisional Patent Publication (PCT) No. 501540/1985 (corresponding to International Patent Publication (PCT) No. WO84/04880).

The human-derived natural collagen membrane is like a medicinal wafer and extremely easily tears and then it is hard to handle it, when used as a monolayer material. Also, when tried to suture, it is easily cut with a thread. Therefore, it is not possible to suture it. In the present invention, in order to have a material having sufficient thickness for easy handling, at least 2 layers of the human-derived natural collagen membrane are laminated by adhering them with an adhesive comprising a collagen solution or a gelatin solution which is decomposed and absorbed in a living body.

In the present invention, at least 2 layers of the human-derived natural collagen membrane are adhered, and from the viewpoint of easy handling, 2 to 10 layers, more preferably 2 to 5 layers, most preferably 4 or 5 layers are adhered to give a laminate. In the case of a monolayer material, it is very hard to handle it as described above. In the case of a laminate of more than 10 layers, it cannot be easily cut and it is hard to handle it. It is particularly easy to handle a laminate of 4 or 5 layers since it maintains its plate-like form in water and has flexibility.

As a collagen which is a raw material of the adhesive comprising a collagen solution, any collagen such as an acid-solubilized collagen, an alkali-solubilized collagen and an enzyme-solubilized collagen may be used as far as the antigenicity is reduced. It is preferred to use a collagen dissolved in hydrochloric acid (about 1 N, pH about 3). As a gelatin which is a raw material of the adhesive comprising a gelatin solution, there may be used a conventionally used gelatin, for example, a general purified gelatin listed in Japanese Pharmacopoeia. A gelatin dissolved in water is preferred.

The collagen solution to be used as the adhesive has a collagen concentration of about 3 % by weight or less, more preferably about 1 to about 3 % by weight, most preferably about 1 % by weight. The gelatin solution to be used as the adhesive has a gelatin concentration of about 1 to about 20 % by weight, and a concentration of about 5 % by weight is preferred from the viewpoint of handling.

As above described, the layers of the human-derived natural collagen membrane which has been collected from a human and purified are laminated by adhering them with the adhesive by a conventionally used immersing or coating method to give a laminate comprising a desired number of layers of the human-derived natural collagen membrane.

In order to keep the adherence of the adhesive with which the layers of the human-derived natural collagen membrane are adhered, in a living body or under a moisture condition, the above laminate is subjected to a crosslinking treatment. As a crosslinking method, there may be mentioned a crosslinking method using γ rays, ultraviolet rays, a crosslinking agent such as glutaraldehyde and epoxy, or heat. The crosslinking method using γ rays cannot be easily controlled, and the crosslinking method using ultraviolet rays does not have a sufficient crosslinking activity. The crosslinking method using a crosslinking agent has a disadvantage due to the toxicity of the crosslinking agent, and also, the crosslinking reaction is liable to proceed uncontrollably, whereby the tissue regeneration-inducibility of a collagen is lost. The crosslinking method using heat is easily controlled and harmless, so that it is preferred to carry out thermal crosslinking. For the thermal crosslinking, the laminate obtained by adhering the layers of the human-derived natural collagen membrane with the above adhesive is heated under high vacuum (about -0.08 mPa or less) at a temperature of about 105 to about 150 °C for about 1 to about 48 hours. At a temperature of less than 105 °C, the crosslinking reaction does not sufficiently proceed. At a temperature of about more than 150 °C, the human-derived natural collagen membrane is denatured. The heating time differs depending on the heating temperature. For example, if the heating temperature is about 105 °C, the heating time as long as about 48 hours will be required. If the heating temperature is about 150 °C, the heating time as short as about 1 hour will be sufficient.

The material for medical use of the present invention obtained as described above may be processed into a desired form, depending on the use. If it is used as a wound dressing material or a visceral wound prosthetic material, it is preferably in the form of a sheet. If it is used as a suture-reinforcement material for an autosuture, it is preferably in the form of a pipe.

If desired, by immersing the obtained material for medical use in a glycerin solution, flexibility can be imparted to the human-derived natural collagen membrane.

The material for medical use of the present invention prepared as described above can be applied to any wound of a living body which does not require a suture.

When the material for medical use of the present invention is used as a wound dressing material, moisture evaporation is naturally controlled and a wound is kept under a dry condition, and therefore bacteria cannot easily proliferate. Furthermore, the healing of the wound is accelerated by the tissue regeneration-inducibility of a collagen. The regenerated tissue under skin goes into the material for medical use with which the wound is covered, and therefore the material does not need to be exchanged. The material for medical use itself has a low antigenicity because it is human-derived.

When the material for medical use of the present invention is used as a suture-reinforcement material for an autosuture, the material for medical use prepared as described above is sutured to have a pipe-like material, an autosuture is equipped with the material, and a suture area is sutured together with the material. It is preferred to suture the material for medical use of the present invention into the form of a pipe by a suturing method by which the suture is easily untied after suturing with the autosuture. The material for medical use of the present invention, together with the other material, may be sutured into the form of a pipe so that the part of the pipe consisting of the material for medical use of the present invention remains in a body and the part of the pipe consisting of the other material is removed after a viscus is sutured with the autosuture. By using the material for medical use of the present invention which is transparent and handled easily as a suture-reinforcement material, an autosuture can be used safely, and the material of the present invention remaining in a body after the suture is safely decomposed and absorbed in the body without causing an immunological response.

When the material for medical use of the present invention is used as a visceral wound prosthetic material, the material of the present invention is applied to an exposed wound of a viscus which has been cut or injured and has lost tunica serosa. Only by applying the material for medical use of the present invention to the wound, it sticks to the surface of the wound by the surface tension of water. If it is required to firmly adhere it, it is adhered with a fibrin paste. The adherence of the fibrin paste continues only for several days. Then, if it is required to more firmly fix the material for medical use of the present invention to the surface of the wound, it may be fix to the surface of the wound by stitching.

By using the material for medical use of the present invention as a visceral wound prosthetic material, the healing of a visceral wound is accelerated, and after the healing of the wound, the material for medical use of the present invention is safely decomposed and absorbed in a living body.

In order to prevent the adhesion of a wound to other living body tissues or viscera in the course of healing when the material for medical use of the present invention is used for various uses described above, one or both surfaces of the material for medical use of the present invention may be further subjected to an adhesion-preventing treatment. For carrying out the adhesion-preventing treatment, a layer comprising a mucopolysaccharide or gelatin is formed on the surface of the material for medical use of the present invention and then the layer comprising a mucopolysaccharide or gelatin is made in the form of gel to sol by mildly subjecting the layer to a thermal dehydration crosslinking treatment. As the mucopolysaccharide, hyaluronic acid, chondroitin sulfate, heparan sulfate or heparin may be used. The layer comprising a mucopolysaccharide or gelatin is used in such an amount that the layer in the form of sol to gel can be retained for about one week in a body, and is formed by forming a layer comprising a mucopolysaccharide or gelatin aqueous solution by means of a conventionally used coating or immersing method, and drying or solidifying it. For the thermal dehydration crosslinking treatment, said layer is heated under high vacuum (about -0.08 mPa or less) at a temperature of about 105 to about 150 °C for about 1 to about 48 hours. At a temperature of less than 105 °C, sufficient crosslinking does not occur, and at a temperature of more than 150 °C, the human-derived natural collagen membrane is denatured. The heating time differs depending on the heating temperature. For example, if the heating temperature is about 105 °C, the heating time as long as about 48 hours will be required. If the heating temperature is about 150 °C, the heating time as short as about 1 hour will be sufficient.

The material for medical use of the present invention subjected to the above adhesion-preventing treatment can be also used as an adhesion-preventing material in various fields, by utilizing adhesion-preventing properties thereof.

The material for medical use of the present invention may be used for various uses in addition to the use as a wound dressing material, a suture-reinforcement material for an autosuture, a visceral wound prosthetic material or an adhesion-preventing material as described above, by utilizing properties thereof.

### EXAMPLES

The present invention is explained in detail by referring to Examples and Comparative examples as described below, but the present invention is not limited by these examples.

### Example 1

A human amnion-derived collagen membrane was obtained from a mass obtained immediately after delivery as an afterbirth by the method described in Japanese Provisional Patent Publication No. 56987/1993. For the enzymatic purification of the amnion, the amnion was immersed in a 0.01 % by weight ficin solution in 0.2 M phosphate buffer (pH 7.4) which also contained 0.02 M cysteine. By laminating and adhering layers of the human amnion-derived collagen membrane with a 2.5 % by weight gelatin aqueous solution, a laminate comprising 2 layers, 4 layers, 6 layers or 8 layers was prepared. The laminates were subjected to a thermal dehydration crosslinking treatment under high vacuum (about -0.08 mPa or less) at 105 °C, 120 °C, 130 °C, 140 °C or 150 °C for 24 hours, respectively. Furthermore, a thermal dehydration crosslinking treatment was carried out by changing the temperature condition and also the heating time between 2 hours and 24 hours.

The materials obtained were immersed in water, and the period until the amnion peeled off was observed. The results are shown in Table 1 and Table 2.

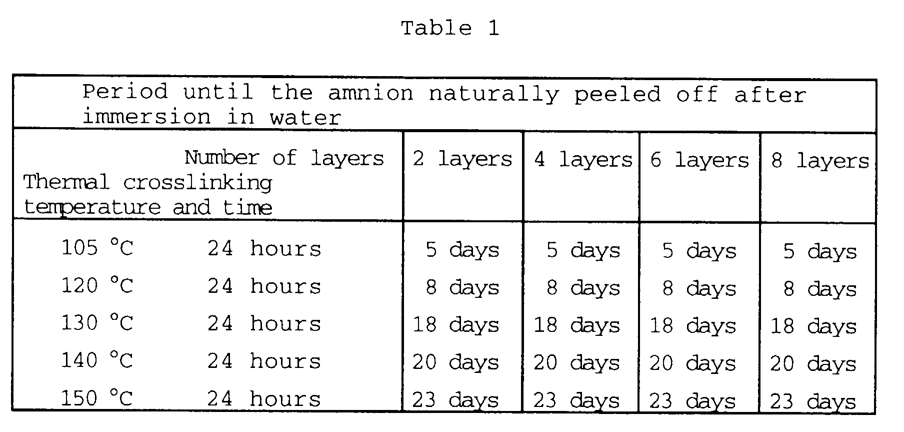

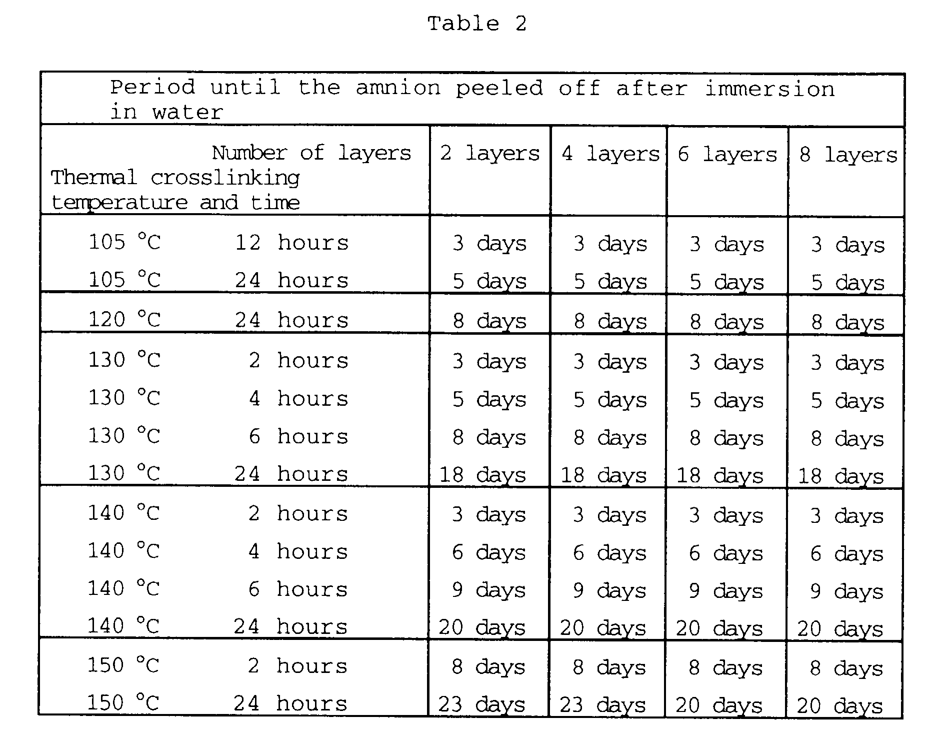

As shown in Table 1 and Table 2, the material for medical use of the present invention prepared under any condition showed that the period until the amnion peeled off was 3 days or more. When the material for medical use of the present invention is used, it is essential that the collagen membrane does not peel off when handled during an operation. The above results showed that the material of the present invention satisfies this requirement.

### Example 2

Laminates were prepared in the same manner as in Example 1 except that a 1 % by weight collagen solution in about 1 N hydrochloric acid (pH about 3) was used as an adhesive. The period until the amnion peeled off in water was observed. The results are shown in Table 3 and Table 4.

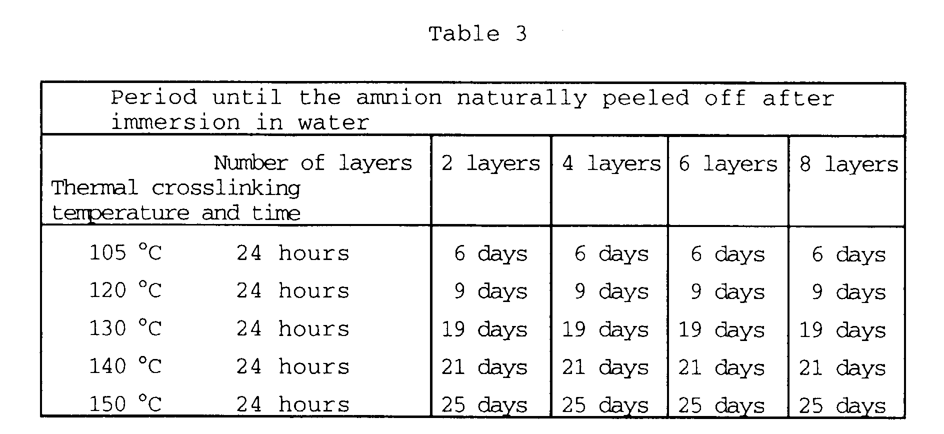

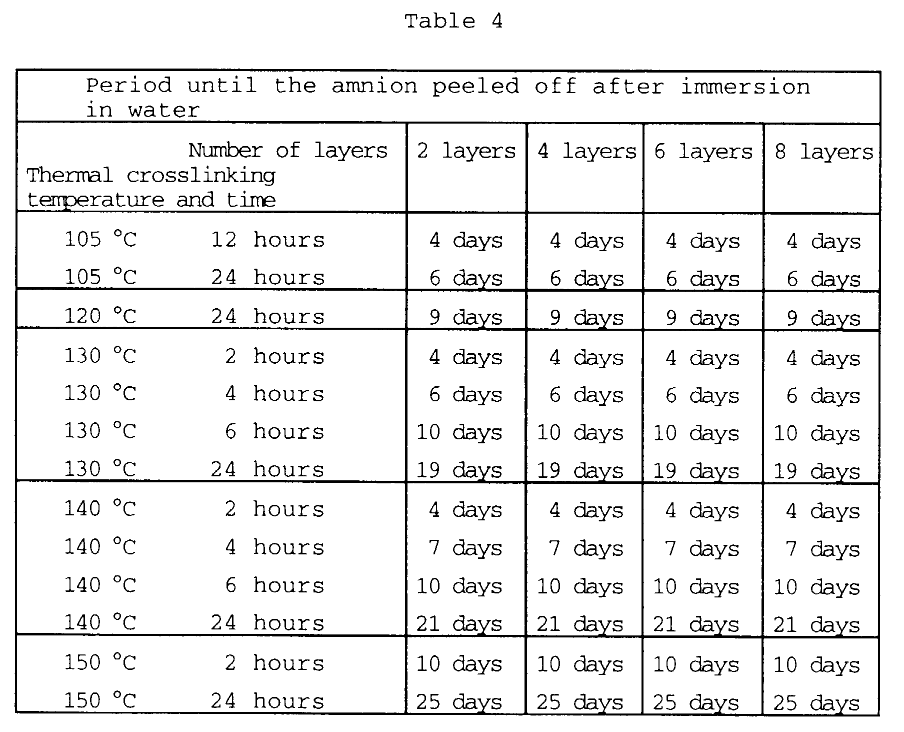

As shown in Table 3 and Table 4, the material for medical use of the present invention prepared under any condition showed a sufficient period until the amnion peeled off.

### Example 3

By using layers of the human amnion-derived collagen membrane obtained by the method described in Example 1 and a 2.5 % by weight gelatin aqueous solution, a laminate of 2 layers, 4 layers, 6 layers or 8 layers was prepared. The laminates were subjected to a thermal crosslinking treatment under high vacuum (about -0.08 mPa or less) at 105 °C for 12 hours to prepare the materials for medical use of the present invention. Then, the tearing strength with a needle when the materials were sutured was measured.

Each material was cut into a patch of 1x4 cm and the both ends were pierced and tied with a wire for suture. The patch was suspended at one end from an exchanger (AIKON, Cpu gauge scale capacity: 500 gf, B-type) and also suspended at the other end from a test stand (AIKON ENGINEERING, MODEL-1356) to measure the tearing strength with a needle under a dry condition and a moisture condition. The results are shown in Table 5.

**Table 5**

| Sample | Peak value of tearing strength with needle under dry condition (g) | Peak value of tearing strength with needle under moisture condition (g) |
|---|---|---|
| 8 layers | 36 to 50 | 47 |
| 6 layers | 35 to 45 | 35 |
| 4 layers | 26 to 38 | 37 |
| 2 layers | 14 to 18 | 12 |

As shown in Table 5, the material for medical use of the present invention prepared under any condition showed such a strength that it can be easily cut with an autosuture.

### Example 4

### Effect on full thickness skin deficit as wound dressing material by laminate

The material comprising 2 layers of the human amnion-derived collagen membrane prepared in the same manner as in Example 3 was applied to full thickness apellous wounds of a pig skin so as to investigate the effect as a wound dressing material.

Six full thickness apellous wounds in square of 3x3 cm² were made on the back of a female juvenile pig. The material comprising 2 layers of the human amnion-derived collagen membrane was applied to two wounds, a swine skin-derived extracted and reconstituted collagen sponge was applied to other two wounds, and nothing was applied to remaining two wounds to be open wounds. The wounds were observed 2 days, 5 days, 7 days, 9 days, 12 days, 16 days and 30 days after the operation.

On the second day, an exudate was observed in the open wounds, and a large amount of an exudate was observed in the wounds to which the swine skin-derived extracted and reconstituted collagen sponge was applied, whereas the wounds to which the material comprising 2 layers of the human amnion-derived collagen membrane was applied remained dry. On the 5th day, all of the wounds were covered with a crust, but an exudate was still observed in the open wounds and the wounds to which the swine skin-derived extracted and reconstituted collagen sponge was applied. The wounds to which the material comprising 2 layers of the human amnion-derived collagen membrane was applied remained dry.

On the 12th to 16th days after the operation, the crusts on the open wounds and the wounds to which the swine skin-derived extracted and reconstituted collagen sponge was applied could be artificially peeled off. On the other hand, the crusts on the wounds to which the material comprising 2 layers of the human amnion-derived collagen membrane was applied could not be peeled off, and when tried to remove the crusts by force, the wounds showed hemorrhage. On the 30th day, all of the wounds had epithelium. The wounds to which the material comprising 2 layers of the human amnion-derived collagen membrane was applied had subcutaneous tissues which were more normal than those of the wounds to which the swine skin-derived extracted and reconstituted collagen sponge was applied and those of the open wounds.

### Example 5

### Effect on split thickness skin deficit as wound dressing material by laminate

The material comprising 2 layers of the human amnion-derived collagen membrane prepared in the same manner as in Example 3 was applied to a split thickness apellous wound of a pig skin so as to investigate the effect as a wound dressing material.

To the control group, a chitin non-woven fabric, an alginic acid salt non-woven fabric, a lyophilized swine dermis layer, a petrolatum-coated knit, a polyurethane membrane or an antimicrobial agent-containing ointment-coated gauze was applied for comparison.

In the experiment, a pig was subjected to split-thickness skin excision in the thickness of 35/1,000 inch by a dermatome on the regions of back, and the above respective materials were applied. The wounds were observed for 10 days after the operation. The material comprising 2 layers of the human amnion-derived collagen membrane closely stuck to the surface of the wound, no exudate under the membrane was observed, no heterologous response as a cellular response was observed, and on the 10th day, the healing of the wound was observed.

With respect to the wounds to which the other materials were applied, an exudate was observed on the surfaces of the wounds, but on the 10th day, the wounds were healed.

### Example 6

The material comprising 4 layers of the human amnion-derived collagen membrane prepared in the same manner as in Example 3 was applied to visceral wounds. Lung, liver, spleen and pancreas of a beagle were partly excised, and the material comprising 4 layers of the human amnion-derived collagen membrane was applied to the sections of the excised viscera so as to observe whether or not the flow out of air, blood, bile juice or pancreatic juice could be stopped.

In any wound, the material comprising 4 layers of the human amnion-derived collagen membrane closely stuck to the section. Also, since said material was transparent, the section could be clearly observed. After the operation, no flow out of blood, bile juice nor pancreatic juice from the wound surfaces was observed, so that it was not required to use a drainage tube for air or body fluid, and the wounds all healed. When the condition of each viscus was examined after 6 weeks, the section of any viscus healed, and the material comprising 4 layers of the human amnion-derived collagen membrane was absorbed and disappeared.

### Comparative example 1

An experiment was carried out in the same manner as in Example 6 except that a polyglycolic acid non-woven fabric (a laminate comprising 4 layers or 6 layers) was used in place of the material comprising 4 layers of the human amnion-derived collagen membrane. When the polyglycolic acid non-woven fabric was applied to the section of each viscus, the fabric could not stick to the section by itself, and it was required to adhere it with a fibrin paste. Also, the section could not be seen through the non-woven fabric due to its opaqueness, so that it was difficult to observe the condition of the adhesion. When each viscus was observed 6 weeks after the operation, the non-woven fabric still remained at the applied area, and an inflammation response was observed around the applied area.

### Example 7

As a suture-reinforcement material for an autosuture when partial excision of a viscus was carried out by using an autosuture, the material comprising 3 layers of the human amnion-derived collagen membrane prepared in the same manner as in Example 3 was sutured into a pipe-like form. Then, the sutured material was put over both handles of an autosuture, and a viscus was subjected to suture-excision so as to observe the reinforcement effect of the material. In particular, lung of a dog was partially suture-excised with the autosuture on which the material comprising 3 layers of the human amnion-derived collagen membrane was put. No leakage of air was observed at the edge of the excised area, even under a pressure of 50 cmH₂O.

When the edge of the excised area of the lung was examined 6 weeks after the operation, the wound showed good healing, and the material comprising 3 layers of the human amnion-derived collagen membrane was absorbed and disappeared.

### Comparative example 2

An experiment was carried out in the same manner as in Example 7 except that a polyglycolic acid non-woven fabric (a laminate comprising 4 layers or 6 layers) was used as a suture-reinforcement material for an autosuture in place of the material comprising 3 layers of the human amnion-derived collagen membrane. The suture with an autosuture itself could be carried out. However, the polyglycolic acid non-woven fabric was too hard, so that it was difficult to cut the non-woven fabric with a cutter. Also, since the non-woven fabric was not transparent, it could not be judged whether or not the lung was properly sutured. Therefore, air leakage could not be controlled in some cases. When the wound was examined 6 weeks after the operation, the non-woven fabric was still present, and inflammation brought about by the presence of the fabric and the high adhesion of the fabric with the surrounding tissues were observed.

### Comparative example 3

An experiment was carried out in the same manner as in Example 7 and Comparative example 2 except that a bovine pericardium crosslinked with glutaraldehyde was used as a suture-reinforcement material for an autosuture. The bovine pericardium was transparent and was not so thick, so that the suture-excision with an autosuture could be carried out. When the section of the lung was observed 6 weeks after the operation, the bovine pericardium was still present intactly, and the high adhesion of the pericardium with the surrounding tissues was observed.

### Example 8

On one surface of the material comprising 3 layers of the human amnion-derived collagen membrane prepared in the same manner as in Example 3, a gel layer of gelatin was formed to examine an adhesion-preventing effect.

One surface of the material comprising 3 layers of the human amnion-derived collagen membrane was coated with a 20 % by weight gelatin aqueous solution to form a gelatin layer having a dry thickness of 5 mm and subjected to a thermal dehydration crosslinking treatment under high vacuum (about -0.08 mPa or less) at 105 °C for 24 hours. Then, the material was immersed *in vitro* in water so as to examine a fusion rate. As a result, it was found that the gel layer of gelatin remained on the surface of the material for about 10 days.

The material comprising 3 layers of the human amnion-derived collagen membrane and having the gel layer formed thereon was sutured to an apellous wound (3x3 cm) of a dog pericardium to observe whether or not the wound adhered. When the wound of the pericardium was observed after 6 weeks, no adhesion was found. Also, the prosthetic material comprising 3 layers of the human amnion-derived collagen membrane disappeared and was substituted by a newly regenerated inherent pericardium.

Since the material for medical use of the present invention comprising a laminate employs a human-derived natural collagen membrane, it has a low antigenicity, does not possibly cause an immunological response and becomes dry immediately after it is applied to a wound, which prevents bacterial proliferation. Also, capillary vessels of a living body extend into the applied material, whereby the regeneration of living body tissues is accelerated. After the material is applied to a living body, the material is decomposed and absorbed, then it does not need to be removed later. Accordingly, the material for medical use of the present invention can be suitably used as a wound dressing material, a suture-reinforcement material for an autosuture or a visceral wound prosthetic material.

## Claims

1. A material for medical use, which comprises at least 2 layers of a human-derived natural collagen membrane, wherein said layers of the human-derived natural collagen membrane are adhered with an adhesive.

2. The material according to Claim 1, wherein the human-derived natural collagen membrane is a human amnion-derived collagen membrane or a human chorion-derived collagen membrane.

3. The material according to Claim 1, which comprises 2 to 10 layers of the human-derived natural collagen membrane.

4. The material according to Claim 2, which comprises 2 to 10 layers of the human amnion-derived collagen membrane or the human chorion-derived collagen membrane.

5. The material according to Claim 4, which comprises 2 to 5 layers of the human amnion-derived collagen membrane or the human chorion-derived collagen membrane.

6. The material according to Claim 5, which comprises 4 or 5 layers of the human amnion-derived collagen membrane or the human chorion-derived collagen membrane.

7. The material according to Claim 1, wherein the adhesive is a collagen solution or a gelatin solution.

8. The material according to Claim 1, which further has a layer which comprises a mucopolysaccharide or gelatin and has been formed in the form of sol to gel by subjecting the layer to a thermal dehydration crosslinking treatment, on the surface thereof.

9. A wound dressing material which comprises the material according to any one of Claims 1 to 8.

10. A suture-reinforcement material for an autosuture, which comprises the material according to any one of Claims 1 to 8.

11. A visceral wound prosthetic material which comprises the material according to any one of Claims 1 to 8.

12. An adhesion-preventing material which comprises the material according to Claim 8.

13. A process for preparing the material according to Claim 1, which comprises adhering at least 2 layers of a human-derived natural collagen membrane with an adhesive to give a laminate and subjecting the laminate to a crosslinking treatment.

14. The process according to Claim 13, wherein the crosslinking treatment is a thermal crosslinking treatment.
